# FASCICULE DE BREVET EUROPEEN

(11) **EP 2 370 066 B1**
(45) Date de publication et mention de la délivrance du brevet: **30.03.2016**
(21) Numéro de dépôt: 09801470.7
(22) Date de dépôt: 01.12.2009
(51) Int. Cl.: A61K 9/48, A23P 10/30

(54) **PROCEDE DE FABRICATION D'UNE SERIE DE CAPSULES, ET SERIE DE CAPSULES ASSOCIEE**
VERFAHREN ZUR HERSTELLUNG VON KAPSELREIHEN UND RELEVANTE KAPSELREIHE
METHOD FOR MANUFACTURING CAPSULE SERIES, AND RELATED CAPSULE SERIES

(30) Priorité: 01.12.2008 FR 0858172
(43) Date de publication de la demande: 05.10.2011
(73) Titulaire: Capsum, 13013 Marseille (FR)
(72) Inventeur: BIBETTE, Jérôme, F-75006 Paris (FR); CHU, Liang-yin, Chengdu 610065 (CN); SANTANACH CARRERAS, Enric, F-75013 Paris (FR); ROYERE, Audrey, F-75005 Paris (FR); BREMOND, Nicolas, F-75005 Paris (FR)
(74) Mandataire: Blot, Philippe Robert Emile
(86) Numéro de dépôt international: PCT/FR2009/052351
(87) Numéro de publication internationale: WO 2010/063937

(56) Documents cités:
- EP-A1- 1 310 229
- WO-A1-2006/136196
- GB-A- 2 192 171
- US-A- 5 330 835
- US-A- 5 650 232
- US-A1- 2007 145 326

## Description

La présente invention concerne un procédé de fabrication d'une série de capsules, selon le préambule de la revendication 1.

De telles capsules, qui comportent un coeur liquide encapsulé par une enveloppe gélifiée sensiblement solide, présentent des applications dans de nombreux domaines techniques.

Ainsi, dans l'industrie alimentaire, ces capsules sont utilisées pour contenir des additifs variés qui permettent d'améliorer les propriétés d'un produit alimentaire, tels que son goût, ou sa durée de conservation.

Dans l'industrie pharmaceutique ou dans l'industrie cosmétique, les capsules précitées sont notamment remplies de produits biologiquement ou cosmétiquement actifs. Elles sont utilisées notamment pour protéger leur contenu et contrôler le relargage du produit qu'elles contiennent

De telles capsules sont aussi utilisées dans des applications en biochimie pour immobiliser des cellules dans des bioréacteurs ou comme cellules artificielles dans des implants.

Dans toutes ces applications, les enveloppes des capsules sont généralement formées d'un matériau biocompatible avec le corps humain. A cet effet, il est connu de former l'enveloppe avec des polymères tels que des polysaccharides, qui sont biocompatibles, biodégradables et dans la plupart des cas non toxiques. Ces polymères peuvent avantageusement passer d'un état liquide en solution à un état notablement plus visqueux pour former un gel assurant une rétention mécanique du liquide contenu dans la capsule.

Parmi ces polysaccharides, les alginates sont utilisés en particulier pour créer des structures coeur-enveloppe dans lesquelles le coeur est liquide.

Toutefois, les procédés de fabrication de capsules avec une morphologie contrôlée (diamètre, taille de l'enveloppe) sont fastidieux à mettre en oeuvre. Ainsi, les techniques actuelles impliquent par exemple de former un noyau précurseur solide et de faire croître couche par couche une écorce de polyélectrolyte autour du noyau.

Une fois l'épaisseur d'écorce souhaitée obtenue, le noyau précurseur solide est dissous et le produit liquide à encapsuler est imprégné à l'intérieur de l'écorce.

Une telle technique est fastidieuse et difficile à mettre en oeuvre pour une production en grande série. Par ailleurs, il est nécessaire de disposer d'une grande quantité de produit à encapsuler pour imprégner le coeur, ce qui n'est pas très économique lorsque ces produits sont onéreux.

On connaît en outre de US 6 056 992 un procédé de fabrication de capsules du type précité, dans lequel une première solution destinée à former le coeur et une deuxième solution qui se dépose autour du coeur sont coextrudées pour former une goutte.

La deuxième solution contient un polymère propre à gélifier thermiquement, qui est mis en contact avec un bain à haute température pour former un gel à la surface de la capsule.

Une telle approche ne donne pas entière satisfaction. En effet, dans le cas où la gélation est induite thermiquement, celle-ci est très lente, ce qui conduit à des épaisseurs d'enveloppes non homogènes et difficiles à contrôler. De plus, une gélification thermique peut ne pas être totalement réversible.

Pour pallier ce problème, il est connu d'utillser des polyélectrolytes sensibles par exemple à une solution contenant des ions polyvalents. Dans ce cas, la réaction est rapide, mais il est difficile voire impossible de former des gouttes comprenant un noyau liquide de premier produit et une enveloppe liquide contenant le polyélectrolyte destiné à gélifier sans observer une séparation de phases.

La mise en oeuvre du procédé par coextrusion est dans ce cas très difficile, notamment pour les alginates.

GB 1 192 171 décrit un procédé de formation de capsules selon le préambule de la revendication 1.

US 2007/0145326**,** WO 2006/136196**,** EP 1 310 229**,** US 5 330835 **et** US 5 650 232 décrivent des procédés de formation de capsules dans lesquelles les capsules sont formées directement à travers un liquide.

Un but de l'invention est donc d'obtenir un procédé de fabrication d'une série de capsules présentant des enveloppes fines et de structure très contrôlée encapsulant un coeur liquide, ce procédé étant simple et efficace à mettre en oeuvre.

A cet effet, l'invention a pour objet un procédé selon la revendication 1.

Le procédé selon l'invention peut comprendre l'une ou plusieurs des caractéristiques des revendications 2 à 8.

L'invention a également pour objet une série de capsules selon la revendication 9.

L'invention a également pour objet l'utilisation d'une série de capsules telle que définie ci-dessus comme unité de dosage d'un produit cosmétique, dermatologique ou parapharmacique.

L'invention a en outre pour objet l'utilisation d'une série de capsules telle que définie ci-dessus comme perle de saveur contenant un produit alimentaire ou comme bombe de douceur destinée à être ajoutée à une boisson.

L'invention a également pour objet l'utilisation d'une série de capsules telle que définie ci-dessus en tant que réservoir pour la croissance de cellules pour réaliser des tests de détection de maladies ou pour former un bioréacteur.

L'invention sera mieux comprise à la lecture qui va suivre, donnée uniquement à titre d'exemple, et faite en se référant aux dessins annexés, sur lesquels :
- la Figure 1 est une vue schématique en coupe suivant un plan vertical médian d'un premier dispositif de fabrication de capsules selon l'invention, lors de la fabrication d'une série de capsules selon l'invention ;
- la Figure 2 est une vue à plus grande échelle, en coupe suivant un plan vertical médian d'une capsule selon l'invention fabriquée par le procédé représenté sur la Figure 1;
- la Figure 3 est une vue analogue à la Figure 1 d'un détail d'un deuxième dispositif de fabrication de capsules selon l'invention;
- la Figure 4 est une vue, prise en coupe suivant un plan vertical médian, d'un troisième dispositif de fabrication de capsules selon l'invention.

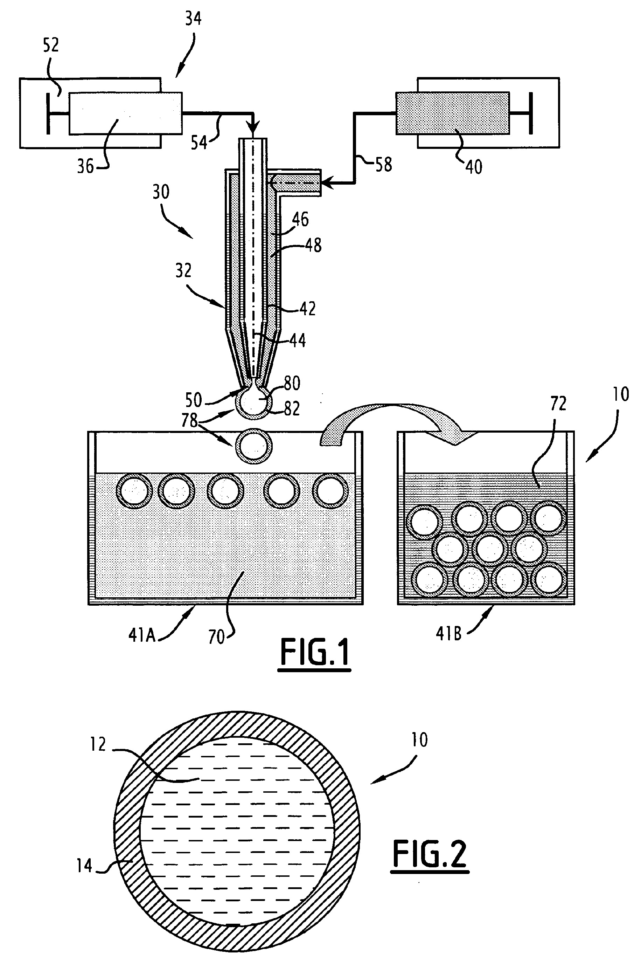

Une première série de capsules 10 selon l'invention est représentée sur la Figure 1. En référence à la Figure 2, chaque capsule 10 comprend un coeur liquide 12 et une enveloppe extérieure gélifiée 14 entourant la totalité de la surface extérieure du coeur 12 pour retenir le coeur liquide 12.

Dans cet exemple, chaque capsule 10 est de forme sphérique et présente avantageusement un diamètre extérieur inférieur à 5 mm et compris notamment entre 1 mm et 3 mm.

Le coeur liquide 12 contient au moins un premier produit choisi avantageusement parmi un produit biologiquement actif, un produit cosmétique, ou un produit comestible propre à être consommé.

Lorsque le premier produit est un produit biologiquement actif, il est choisi avantageusement parmi les anticoagulants, les anti-thrombogéniques, les agents antimitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques.

En variante, le coeur liquide 12 contient des agents réactifs tels que des protéines ou des réactifs destinés à former un bioréacteur, ou à former des cellules artificielles pour des implants.

Un produit cosmétique pouvant être contenu dans le coeur est par exemple cité dans la Directive 93/35/CEE du Conseil datée du 14 juin 1993. Ce produit est par exemple une crème, une émulsion, une lotion, un gel et une huile pour la peau (mains, visage, pieds, etc.), un fond de teint (liquide, pâte) une préparation pour bains et douches (sels, mousses, huiles, gels, etc.), un produit de soins capillaires (teintures capillaires et décolorants), un produit de nettoyage (lotions, poudres, shampoings), un produit d'entretien pour la chevelure (lotions, crèmes, huiles), un produit de coiffage (lotions, laques, brillantines), un produit pour le rasage (savons, mousses, lotions, etc.), un produit destiné à être appliqué sur les lèvres , un produit solaire, un produit de bronzage sans soleil, un produit permettant de blanchir la peau, un produit antirides.

Les produits comestibles propres à être consommés par un être humain ou par un animal sont avantageusement des purées de légumes ou de fruits telles que la purée de mangue, de la purée de poire, de la purée de coco, de la crème d'oignons, de poireaux, de carottes, ou d'autres préparations pouvant mélanger plusieurs fruits ou légumes. En variante, il s'agit d'huiles telles qu'une huile alimentaire, du type huile d'olive, huile de soja, huile de grains de raisin, huile de tournesol, ou toute autre huile extraite des végétaux.

Le coeur 12 se présente avantageusement sous la forme d'un premier produit liquide pur, d'une solution du ou de chaque premier produit dans un solvant liquide, d'une dispersion telle qu'une émulsion ou une suspension du ou de chaque premier produit dans un liquide.

La viscosité du coeur liquide 12 est inférieure à 2000 mPa.s.

Le coeur liquide 12 est à base d'une phase majoritairement aqueuse ou au contraire d'une phase majoritairement huileuse.

L'enveloppe gélifiée 14 des capsules 10 selon l'invention comprend un gel contenant de l'eau et au moins un polyélectrolyte réactif aux ions multivalents. Selon l'invention, l'enveloppe 14 contient en outre un agent tensioactif résultant de son procédé de fabrication, comme on va le décrire en détail plus bas. Par « polyélectrolyte réactif aux ions polyvalents », on entend, au sens de la présente invention un polyélectrolyte susceptible de passer d'un état liquide dans une solution aqueuse à un état gélifié sous l'effet d'un contact avec une solution gélifiante contenant des ions multivalents tels que des ions d'un métal alcalino-terreux choisis par exemple parmi les ions calcium, les ions baryum, les ions magnésium.

Dans l'état liquide, les chaînes individuelles de polyélectrolyte sont sensiblement libres de s'écouler les unes par rapport aux autres. Une solution aqueuse de 2% en masse de polyélectrolyte présente alors un comportement purement visqueux aux gradients de cisaillement caractéristiques du procédé de mise en forme. La viscosité de cette solution à cisaillement nul est entre 50 mPa.s et 10000 mPa.s avantageusement entre 3000 mPa.s et 7000 mPa.s.

Les chaînes individuelles de polyélectrolyte dans l'état liquide présentent avantageusement une masse molaire supérieure à 65000 g/moles.

Dans l'état gélifié, les chaînes individuelles de polyélectrolyte forment, avec les ions multivalents, un réseau tridimensionnel cohérent qui retient le coeur liquide et empêche son écoulement. Les chaînes individuelles sont retenues les unes par rapport aux autres et ne peuvent pas s'écouler librement les unes par rapport aux autres. Dans cet état, la viscosité du gel formé est infinie. De plus le gel a un seuil de contrainte à l'écoulement. Ce seuil de contrainte est supérieur à 0,05 Pa. Le gel possède également un module d'élasticité non-nul et supérieur à 35 kPa.

Le gel tridimensionnel de polyélectrolyte contenu dans l'enveloppe 14 emprisonne de l'eau et l'agent tensioactif. La teneur massique du polyélectrolyte dans l'enveloppe 12 est par exemple comprise entre 0,5 % et 5 %.

Le polyélectrolyte est de préférence un polymère biocompatible inoffensif pour le corps humain. Il est par exemple produit biologiquement.

Avantageusement, il est choisi parmi les polysaccharides, polyélectrolytes de synthèse à base d'acrylates (polyacrylate de sodium, de lithium, de potassium ou d'ammonium, ou polyacrylamide), de polyélectrolytes de synthèse à base de sulfonates (poly(styrène sulfonate) de sodium, par exemple). Plus particulièrement, le polyélectrolyte est choisi parmi un alginate d'alcalino-terreux, tel qu'un alginate de sodium ou un alginate de potassium, une gellane ou une pectine.

Les alginates sont produits à partir d'algues brunes appelées « laminaires », désignées par le terme anglais « sea weed ».

De tels alginates présentent avantageusement une teneur en α-L-guluronate supérieure à environ 50%, de préférence supérieure à 55%, voire supérieure à 60%.

L'agent tensioactif est avantageusement un tensioactif anionique, un tensioactif nonionique, un tensioactif cationique ou un mélange de ceux-ci. La masse moléculaire de l'agent tensioactif est comprise entre 150 g/mol et 10000 g/mol, avantageusement entre 250 g/mol et 1500 g/mol.

Dans le cas où le tensioactif est un tensioactif anionique, il est par exemple choisi parmi un alkylsulfate, un alkyle sulfonate, un alkylarylsulfonate, un alkylphosphate alcalin, un dialkylsulfosuccinate, un sel d'alcalino-terreux d'acides gras saturés ou non. Ces tensioactifs présentent avantageusement au moins une chaîne hydrocarbonée hydrophobe présentant un nombre de carbones supérieur à 5, voire 10 et au moins un groupement anionique hydrophile, tel qu'un sulfate, un sulfonate ou un carboxylate lié à une extrémité de la chaîne hydrophobe.

Dans le cas où le tensioactif est un tensioactif cationique, il est par exemple choisi parmi un sel d'halogénure d'alkylpyridium ou d'alkylammonium comme le chlorure ou le bromure de n-éthyldodecylammonium, le chlorure ou le bromure de cétylammonium (CTAB). Ces tensioactifs présentent avantageusement au moins une chaîne hydrocarbonée hydrophobe présentant un nombre de carbones supérieur à 5, voire 10 et au moins un groupement cationique hydrophile, tel qu'un cation d'ammonium quaternaire.

Dans le cas où le tensioactif est un tensioactif nonionique, il est par exemple choisi parmi des dérivés polyoxyéthylénés et/ou polyoxypropylénés des alcools gras, des acides gras, ou des alkylphénols, des arylphénols, ou parmi des alkyls glucosides, des polysorbates, des cocamides.

La teneur massique en agent tensioactif dans l'enveloppe est supérieure à 0,001% et est avantageusement supérieure à 0,1 %.

Dans cet exemple, l'enveloppe 14 est constituée exclusivement de polyélectrolyte, d'agent tensioactif, et d'eau. La somme des teneurs massiques en polyélectrolyte, en agent tensioactif, et en eau est alors égale à 100%.

Le procédé de fabrication des capsules 10 selon l'invention est mis en oeuvre dans un dispositif de fabrication 30 selon l'invention, représenté sur la figure 1.

Le dispositif de fabrication 30 comprend une double enveloppe 32 pour coextruder une série de gouttes destinées à former des capsules 10, des moyens 34 d'amenée dans la double enveloppe 32 d'une première solution 36 destinée à former le coeur liquide 12, et des moyens 38 d'amenée dans la double enveloppe 32 d'une deuxième solution 40 destinée à former l'enveloppe gélifiée 14.

Le dispositif 30 comprend en outre un bain 41A de gélification disposé sous la double enveloppe 32 et un bain 41 B de rinçage et de stockage.

De manière connue, la double enveloppe 32 comprend un tube intérieur 42 délimitant une chambre centrale 44 de circulation de la première solution 36, et un tube extérieur 46 délimitant, avec le tube intérieur 42, une chambre annulaire 48 de circulation de la deuxième solution 40.

Le tube intérieur 42 et le tube extérieur 46 s'étendent coaxialement le long d'un axe vertical A-A'. Ils débouchent vers le bas par une ouverture 50 de formation de chaque goutte.

Le tube intérieur 42 présente avantageusement un diamètre supérieur à 0,5 mm et sensiblement compris entre 0,6 mm et 2 mm.

Le tube extérieur 46 présente un diamètre supérieur au tube intérieur 42 d'au moins 0,2 mm, avantageusement d'au moins 0,4 mm. Le diamètre maximal du tube extérieur 46 est inférieur à 5 mm.

Chaque tube 42, 46 présente une section transversale convergente vers le bas au voisinage de l'ouverture 50.

Les moyens 34 d'amenée de la première solution 36 comprennent une première pompe 52 de distribution de la première solution 36, raccordée hydrauliquement en aval à la chambre centrale 44 par une première conduite 54 de convoyage.

La première pompe 52 est avantageusement une pompe seringue propre à commander un débit d'injection Q1 donné de la première solution 36 dans la chambre centrale compris entre 1 ml/h et 120 ml/h, de préférence dans la gamme comprise entre 50 ml/h et 80 ml/h.

Les moyens d'amenée 38 de la deuxième solution 40 comprennent une deuxième pompe 56 de distribution de la deuxième solution 40, raccordée en aval à la chambre annulaire 48 par l'intermédiaire d'une deuxième conduite 58 de convoyage.

La deuxième pompe 56 est avantageusement une pompe seringue propre à commander le débit d'injection Q2 de deuxième solution 40 dans la chambre annulaire 48 que ce débit Q2 soit compris entre 0,005 fois et 0,2 fois le débit Q1 commandé par la première pompe 52.

La première solution 36 est formée par le ou chaque premier produit liquide pur, une solution du ou de chaque premier produit dans un solvant liquide, une dispersion telle qu'une émulsion ou une suspension du ou de chaque premier produit dans un liquide telle que décrite plus haut.

La deuxième solution contient le polyélectrolyte liquide propre à gélifier destiné à former l'enveloppe liquide 12, de l'eau et en outre, selon l'invention, au moins un agent tensioactif permettant la réalisation des capsules 10.

Le polyélectrolyte a été décrit en détail plus haut et ne sera pas redécrit. Il est totalement dissous dans l'eau formant la deuxième solution.

La teneur massique du polyélectrolyte dans la deuxième solution est supérieure à la teneur massique du polyélectrolyte dans l'enveloppe 14. Dans la deuxième solution, cette teneur massique est supérieure à 0,1% et est par exemple comprise entre 0,1% et 5% en poids de la deuxième solution.

Le ou chaque agent tensioactif a été décrit plus haut. La teneur massique en tensioactif est comprise entre 0,01% et 1% en masse de la masse totale de la deuxième solution.

Si la composition du coeur liquide 12 est majoritairement aqueuse, la concentration en agent tensioactif dans la deuxième solution est avantageusement comprise entre 0,01% et 0,5% en masse. Si la composition du coeur liquide 12 est majoritairement huileuse, la concentration en agent tensioactif est comprise entre 0,1% et 0,5% en masse.

Avantageusement, la concentration en masse de tensioactif est d'environ 0,03% pour un coeur liquide 12 aqueux et est d'environ 0,15% pour un corps liquide 12 huileux.

La deuxième solution est préparée par dissolution de l'agent tensioactif dans la quantité d'eau nécessaire pour former la deuxième solution. Puis, le polyélectrolyte est ajouté à la solution de tensioactif dans l'eau et est mélangé à l'aide d'un barreau magnétique pendant un temps donné, par exemple au moins 24 heures, à température ambiante.

Le bain 41A contient une solution gélifiante 70. Cette solution 70 est par exemple une solution aqueuse d'un réactif de type XₙIₘ où X est avantageusement un ion halogénure tel qu'un ion chlorure, un ion bromure, un ion iodure ou un ion fluorure, et I est avantageusement un cation multivalent d'un alcalino-terreux tel que le calcium, le magnésium, ou le baryum, et n et m sont supérieurs ou égaux à 1.

Les ions multivalents présents dans la solution gélifiante 70 ainsi formée sont propres à réagir avec le polyélectrolyte pour former des liaisons entre les différentes chaînes de polyélectrolyte présentes dans la deuxième solution, lorsque la deuxième solution entre en contact avec la solution gélifiante 70.

Dans le cas où le polyélectrolyte est un alginate de sodium (NaAlg), et où le réactif et le chlorure de calcium, la réaction qui se produit est la suivante :

2NaAlg + Ca Cl₂ → Ca(Alg)₂ ↘ + 2NaCl

La concentration en réactif dans la solution de gélification est avantageusement comprise entre 5 % et 20 % en masse.

Le bain 41A est disposé en dessous et à l'écart de l'ouverture 50, de sorte que les gouttes formées par coextrusion dans la double enveloppe 32 tombent spontanément par gravité à travers un volume d'air dans la solution gélifiante 70 où elles sont immergées.

Le bain de rinçage 41 B comprend une solution de rinçage et de stockage constituée essentiellement d'eau.

Un premier procédé de formation de capsules 10 selon l'invention va maintenant être décrit.

Ce procédé comprend une étape de formation d'une goutte 78 comprenant un noyau 80 de première solution et une pellicule externe 82 de deuxième solution par coextrusion dans le dispositif 32. Le procédé comprend ensuite une étape de trempage de la goutte 78 dans le bain de gélification 41A, suivie d'une étape de rinçage/stockage dans le bain de rinçage 41 B.

Initialement, la première solution 36 et la deuxième solution 40 sont préparées comme décrit plus haut avec les charges pondérales en polyélectrolyte et en tensioactif décrites plus haut.

Elles sont ensuite introduites respectivement dans les pompes, 52, 56 qui sont raccordées à la double enveloppe 32 respectivement par la première conduite 54 et par la deuxième conduite 58.

Puis, la première pompe 52 est activée pour convoyer un flux continu de première solution 36 à travers la chambre centrale 44 avec un débit calibré Q1 compris avantageusement entre 10 ml par heure et 80 ml par heure, comme on l'a vu plus haut.

La deuxième pompe 56 est activée pour convoyer simultanément un flux continu de deuxième solution 40 à travers l'espace annulaire 48 à un débit Q2 commandé par exemple entre 0,005 fois et 0,2 fois le débit Q1 de première solution 36.

Le réglage relatif et indépendant des débits Q1 et Q2 permet de commander l'épaisseur de l'enveloppe 14 indépendamment du diamètre extérieur de la capsule 10.

En réduisant significativement le débit Q2, il est par ailleurs possible d'obtenir des capsules 10 avec une enveloppe gélifiée 14 de très faible épaisseur, notamment inférieure à 0.5% du diamètre de la capsule 10, grâce à la présence de l'agent tensioactif dans la deuxième solution 40.

Au niveau de l'ouverture 50, une goutte 78 sensiblement sphérique se forme progressivement avec un noyau 80- constitué exclusivement de première solution et une pellicule fine 82 de deuxième solution entourant totalement la surface extérieure du noyau 80.

Le noyau 80 est constitué exclusivement de première solution 36. Dans la pellicule 82, le polyélectrolyte est maintenu dans son état liquide comme dans la deuxième solution.

Lorsque le poids de la goutte 78 est supérieur à sa force de retenue par capillarité sur les tubes de la double enveloppe 32, la goutte 78 se détache de la double enveloppe 32 par gravité et tombe dans le bain de gélification 41A.

La pellicule 82 est alors au contact de la solution gélifiante. Au contact des ions multivalents provenant du réactif gélifiant, les chaînes individuelles de polyélectrolyte présentes dans la pellicule 82 se raccordent les unes aux autres pour former un réseau réticulé qui emprisonne de l'eau et au moins partiellement de l'agent tensioactif contenu dans la deuxième solution.

Une enveloppe gélifiée 14, propre à retenir le coeur liquide 12 de première solution liquide est ainsi formée. Cette enveloppe 14 présente une tenue mécanique propre, c'est-à-dire qu'elle est capable d'entourer totalement le coeur liquide 12 et de retenir le liquide présent dans ce coeur 12 pour l'empêcher de diffuser à travers l'enveloppe 14, notamment lorsque la capsule 10 est disposée dans un gaz tel que l'air ambiant.

De manière très surprenante, la présence d'agent tensioactif dans la deuxième solution favorise significativement la formation des capsules 10 en empêchant notamment la pellicule 82 de subir une séparation de phase préjudiciable lors de son trempage dans la solution de gélification 70.

Puis, une autre goutte 78 se forme alors à l'extrémité inférieure 50 de la double enveloppe 32 et les étapes du procédé sont alors identiques à celles décrites précédemment.

Une fois les capsules 10 formées, elles sont transférées dans la solution de rinçage 72 en vue de leur stockage. Les capsules 10 ainsi formées stockent donc de manière étanche des composés divers comme des produits biologiquement actifs, des protéines, des produits cosmétiques, ou des produits comestibles destinés à être consommés par un être humain ou un animal.

Lors de l'utilisation des capsules 10, l'enveloppe 12 est rompue par cisaillement, ou par écrasement mécanique, ou par chélation des ions multivalents, à l'aide d'un sel adapté tel que l'EDTA, dans le cas où on utilise des ions calcium pour former le gel de l'enveloppe. Cette rupture permet de récupérer le premier produit présent dans le coeur 12.

Dans une variante d'utilisation, les capsules récupérées sont plongées dans un liquide pour gonfler, puis éclater par contrôle de la pression osmotique à travers l'enveloppe gélifiée.

L'ouverture de l'enveloppe 14 libère alors le coeur liquide 12. Ceci s'applique notamment à des capsules 10 contenant un sirop.

Pour faire gonfler la capsule 10, un polymère tel qu'un polyéthylène glycol de masse moléculaire supérieure à 5 000 g/mol est ajouté au coeur.

En variante, la pression osmotique est contrôlée pour rétracter les capsules 10 et faire diminuer leur diamètre. Il est alors possible de sécher ou de lyophiliser les capsules 10.

Dans une variante, des particules magnétiques peuvent être ajoutées dans la première solution 36 ou/et dans la deuxième solution 40, pour modifier la manipulation des oeufs.

En variante encore, des paillettes sont ajoutées dans la première solution 36 et/ou dans la deuxième solution 40 pour engendrer un effet optique sur les capsules 10.

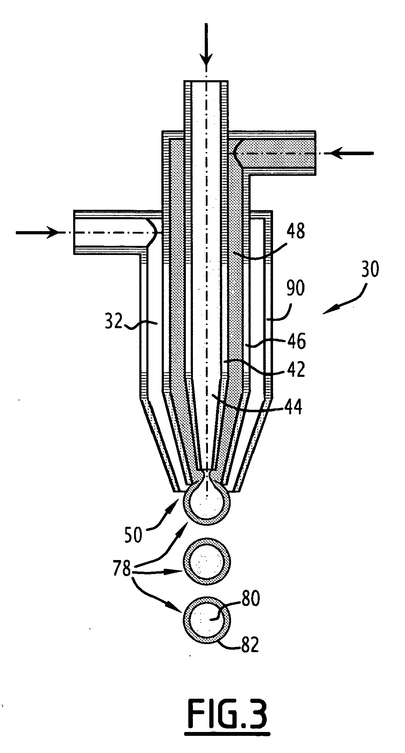

Une variante de dispositif de fabrication 30 de capsules 10 est illustrée par la Figure 3. A la différence du dispositif représenté sur la Figure 1, ce dispositif comprend, autour de la double enveloppe 32, une enveloppe externe 90 d'injection de gaz s'étendant annulairement autour et à l'écart du tube extérieur 46.

L'enveloppe externe 90 débouche axialement autour de l'ouverture 50. Elle est raccordée à une source de gaz sous pression pour créer, autour de la goutte 78 en formation, un flux de gaz dirigé vers le bas.

Le débit de ce flux de gaz peut être réglé pour commander la taille des gouttes 78 formées à la sortie de la double enveloppe 32.

Le procédé de fabrication de capsule 10 utilisant le dispositif 30 selon la Figure 3 diffère juste du procédé mis en oeuvre avec le dispositif 30 de la Figure 1 en ce que la taille des gouttes 78 peut être réglée en réglant le flux de gaz.

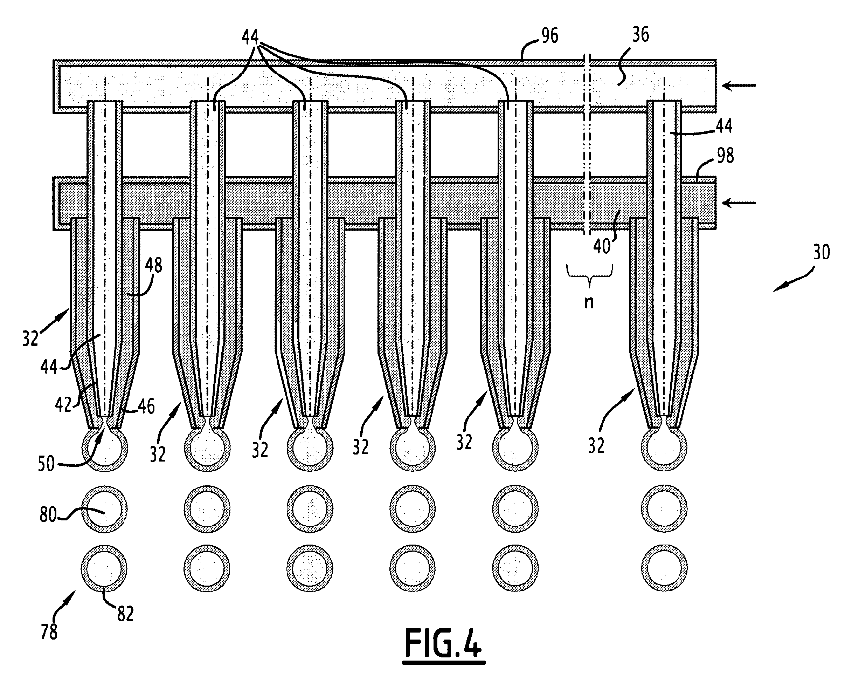

La Figure 4 illustre un troisième dispositif 30 de formation de capsules qui comprend une pluralité de double enveloppes 32 disposées en parallèle les unes à côté des autres. Les moyens d'amenée 34 de la première solution 36 comprennent, à l'extrémité de la première conduite 54 un distributeur commun 96 de première solution, dans lequel débouche chaque chambre centrale 44 des double enveloppes 32 montées en parallèle.

Les moyens d'amenée 38 de la deuxième solution 40 comprennent un distributeur commun 98 de deuxième solution 40 débouchant dans chaque chambre annulaire 48 des doubles enveloppes 32 montées en parallèle. Ainsi, ce dispositif 30 permet de former en parallèle un nombre de gouttes égal au nombre de double enveloppes 32 montées en parallèle ce qui augmente la productivité globale du dispositif.

Dans une variante, dans le cas où le coeur 12 se présente comme une émulsion, cette émulsion peut être fabriquée par coextrusion au sein d'un tube additionnel placé dans le tube intérieur 42 lors de la fabrication de la capsule 10.

Il est ainsi possible de former des coeurs 12 contenant par exemple de la vinaigrette.

Des exemples de compositions de première solution et de deuxième solution ayant été utilisées pour former avec succès des capsules 10 selon l'invention sont décrites dans le tableau ci-dessous, dans lequel tous les pourcentages sont des pourcentages massiques. Le solvant de la deuxième solution est de l'eau. Le PEG est un polyéthylène glycol dont la masse molaire est donne aussi dans le tableau.

Le SDS est du sodium dodécyl sulfate (tensioactif anionique), le CTAB est du Bromure de Cetyl Trimethylammonium (tensioactif cationique), le Tween 20 est du monolaurate de sorbitane polyoxyéthyléné (tensioactif non ionique) et le Tween 80 est du sorbitane mono-oléate polyoxyéthylène (tensioactif non ionique).

| **Composition première solution pour le coeur** | **Composition deuxième solution pour l'enveloppe** |
|---|---|
| | |

| | **AVEC SDS** |
|---|---|
| Eau milliQ | 2% alginate de sodium, 0.03% SDS |
| Eau + 0.5% PEG20000 g/mol | 1.5% alginate de sodium, 0.03% SDS |
| Eau + 0.5% PEG20000 g/mol | 2% alginate de sodium, 0.03% SDS |
| Eau + 0.5% PEG20000 g/mol | 3% alginate de sodium, 0.03% SDS |
| Eau + 1% PEG 20000 g/mol | 2% alginate de sodium, 0.03% SDS |
| Eau + 2% PEG 20000 g/mol | 2% alginate de sodium, 0.03% SDS |
| Eau + 4% PEG 20000 g/mol | 2% alginate de sodium, 0.03% SDS |
| Eau + 0.5% PEG 35000 g/mol | 2% alginate de sodium, 0.03% SDS |
| Eau + 15% PEG 35000 g/mol | 2% alginate de sodium, 0.03% SDS |
| Eau + 20% PEG 35000 g/mol | 2% alginate de sodium, 0.03% SDS |
| *Purée de mangue* 80% purée de mangue+20% sirop à 15% sucre | 2% alginate de sodium, 0.03% SDS |
| *Purée de poire* 80% purée de poire + 20% sirop à 15% sucre | 2% alginate de sodium, 0.03% SDS |
| *Solution de poudre de cacao* 10% de poudre de cacao+90% sirop à 30% sucre | 2% alginate de sodium, 0.03% SDS |
| Huile d'olive | 2% alginate de sodium, 0.15% SDS |
| Huile d'olive + 2% basilic mixé | 2% alginate de sodium, 0.15% SDS |
| Huile de soja | 2% alginate de sodium, 0.15% SDS |
| Huile de pépin de raisin | 2% alginate de sodium, 0.15% SDS |
| Huile de pépin de raisin | 0.5% alginate de sodium, 0.15% SDS |
| Hexadecane | 2% alginate de sodium, 0.15% SDS |
| | |

| | **AVEC CTAB** |
|---|---|
| Eau milliQ | 2% alginate de sodium, 0.02% CTAB |
| Eau milliQ | 2% alginate de sodium, 0.03% CTAB |
| | |

| | **AVEC TWEEN** |
|---|---|
| Eau MilliQ | 2% alginate de sodium, 50mM Tween 20 |
| Eau MilliQ | 2% alginate de sodium, 53mM Tween 80 |

Un exemple de mode opératoire pour la préparation des capsules est le suivant :

### Formation de la deuxième solution :

### Composition pour des capsules avant un coeur aqueux en utilisant un tensioactif anionique :

On prépare 20 g d'alginate de sodium, 1 000 g d'eau et 0,3 g de SDS. Le SDS et l'alginate de sodium sont ensuite ajoutés dans la solution. La solution est mélangée pendant au moins 24 heures pour s'assurer que l'alginate est complètement dissous et que la solution est homogène.

### Composition pour des capsules avec un coeur huileux en utilisant un tensioactif anionique :

On prépare 20 g d'alginate de sodium, 1 000 g d'eau, et 1,5 g de SDS. Le SDS est dissous dans l'eau et l'alginate de sodium est ensuite ajouté. La solution est mélangée pendant au moins 24 heures pour s'assurer que l'alginate est complètement dissous et que la solution est homogène.

### Composition pour des capsules avec un coeur aqueux en utilisant un tensioactif cationique :

On prépare 20 g d'alginate de sodium, 1 000 g d'eau et 0,36 g de CTAB. Le CTAB est dissous dans l'eau et l'alginate de sodium est ensuite ajouté. La solution est mélangée pendant au moins 24 heures pour s'assurer que l'alginate est totalement dissous et que la solution est homogène.

### Composition pour des capsules présentant un coeur huileux en utilisant un tensioactif cationique :

On prépare 20 g d'alginate de sodium, 1 000 g d'eau et 1,8 g de CTAB. Le CTAB est dissous dans l'eau et l'alginate de sodium est ensuite ajouté. La solution est mélangée pendant au moins 24 heures pour s'assurer que l'alginate est totalement dissous et que la solution est homogène.

### Préparation de la première solution

La solution du coeur peut être soit aqueuse, soit huileuse. Elle peut contenir une ou plusieurs phases.

### Bain de solution gélifiante saline

Ce bain peut être préparé à base de 200 g de chlorure de calcium dissous dans 1 000 g d'eau. En variante, il peut être préparé à base de 50 g de pentahydrate de lactate de calcium dissous dans 1 000 g d'eau.

Dans une variante, les capsules 10 préparées par le procédé selon l'invention ne présentent pas de quantité détectable d'agent tensioactif de la deuxième solution.

Le procédé de formation des capsules 10 tel qu'il est décrit plus haut permet d'obtenir des capsules de taille uniforme. Ceci permet de les utiliser comme unité de dosage dans le domaine cosmétique, dermatologique ou dans la parapharmacie.

Ainsi, il est possible de prévoir un traitement cosmétique dans lequel on dose un nombre de capsules à appliquer sur la peau ou à ingérer.

Le nombre de capsules à utiliser, la fréquence d'application de ces capsules ou d'ingestion de ces capsules et la durée du traitement peuvent varier en fonction des caractéristiques de la personne qui subit le traitement.

Dans ce même domaine technique, les capsules 10 peuvent être utilisées avec un coeur 12 qui comprend une crème hydratante pour la peau, des traitements capillaires divers, du gloss pour les lèvres.

Ainsi, il est possible d'éclater une capsule 10 contenant du gloss entre les lèvres d'un utilisateur, le gloss se répartissant en frottant les lèvres l'une contre l'autre.

Dans ce type d'application, des paillettes peuvent être ajoutées dans le coeur 12 ou dans l'enveloppe 14 de la capsule pour obtenir des effets optiques comme une brillance, des reflets, une irradiance.

Les capsules 10 selon l'invention peuvent également être utilisées dans le domaine agroalimentaire pour obtenir des perles de saveur.

Ainsi, les capsules formées contenant un produit alimentaire peuvent être utilisées par exemple pour former un caviar synthétique.

Dans l'agroalimentaire, les capsules 10 peuvent être utilisées aussi comme des bombes de douceur en étant ajoutées à des boissons. Les capsules qui contiennent par exemple un polymère ou un autre produit permettant de les faire gonfler ou dégonfler par pression osmotique permet de larguer un produit contenu dans le coeur 12 dans la boisson ou dans un plat liquide. Ainsi, par gonflement ou dégonflement de la capsule, il est possible de la faire exploser pour libérer le produit contenu dans le coeur 12.

Les capsules 10 selon l'invention peuvent également être utilisées dans le domaine biotechnologique en tant que réservoir pour la croissance de cellules, pour réaliser des tests biologiques de détection de maladie.

Il est ainsi possible d'utiliser des capsules 10 selon l'invention pour faciliter le criblage dans différentes conditions d'environnement.

En outre, l'ajout de particules magnétiques dans l'enveloppe 14 permet d'immobiliser des cellules dans le coeur 12 de la capsule 12 pour former un bioréacteur.

## Revendications

1. Procédé de fabrication d'une série de capsules (10), chaque capsule (10) comprenant un corps liquide (12) contenant au moins un premier produit et une enveloppe gélifiée (14) encapsulant totalement le coeur (12), le procédé comprenant les étapes suivantes :
- convoyage séparé dans une double enveloppe (32) d'une première solution (36) liquide contenant le premier produit et d'une deuxième solution (40) liquide contenant un polyélectrolyte liquide propre à gélifier;
- formation, à la sortie (50) de la double enveloppe (32), d'une série de gouttes (78), chaque goutte (78) comprenant un noyau central (80) formé de première solution (36) et une pellicule périphérique (82) formée de deuxième solution et recouvrant totalement le noyau central (80);
- immersion de chaque goutte (78) dans une solution gélifiante (70) contenant un réactif propre à réagir avec le polyélectrolyte de la pellicule (82) pour le faire passer d'un état liquide à un état gélifié et former l'enveloppe gélifiée (14), le noyau central (80) formant le coeur liquide (12) ;
- récupération des capsules (10) formées dans lequel les gouttes (78) formées par coextrusion dans la double enveloppe (32) tombent par gravité à travers un volume d'air dans la solution gélifiante (70).;
**caractérisé en ce que** la deuxième solution (40) contient au moins un agent tensioactif avant son contact avec la première solution (36), et **en ce que** le rapport du débit de la première solution (36) au débit de la deuxième solution (40) à la sortie de la double enveloppe (32) est compris entre 1 et 200, avantageusement entre 10 et 200, l'enveloppe gélifiée (14) présentant une épaisseur comprise entre 0,1% et 10%, avantageusement entre 0, 1% et 2% du diamètre de la capsule (10), après récupération des capsules formées..

2. Procédé selon la revendication 1, **caractérisé en ce que** le ou chaque agent tensioactif est choisi parmi un agent tensioactif anionique, un agent tensioactif cationique, un agent tensioactif non ionique ou leurs mélanges.

3. Procédé selon la revendication 2, **caractérisé en ce que** l'agent tensioactif est choisi parmi un alkylsulfate, un alkyle sulfonate, un alkylarylsulfonate, un alkylphosphate alcalin, un dialkylsulfosuccinate, un sel d'alcalino-terreux d'acides gras saturés ou non, un sel d'halogénure d'alkylpyridium ou d'alkylammonium comme le chlorure ou le bromure de n-éthyldodecylammonium, le chlorure ou le bromure de cétylamonium, des dérivés polyoxyéthylénés et/ou polyoxypropylénés des alcools gras, des acides gras ou des alkylphénols, ou parmi des arylphénols, des alkyls glucosides, des polysorbates, des cocamides ou leurs mélanges.

4. Procédé selon la revendication 3, **caractérisé en ce que** le pourcentage massique total en agent tensioactif dans la deuxième solution (40) est supérieur à 0,01% et est avantageusement compris entre 0,01% et 0,5% en masse.

5. Procédé selon l'une quelconque des revendications précédentes, **caractérisé en ce que** le ou chaque poyélectrolyte est un polyélectrolyte réactif aux ions multivalents, notamment un polysaccharide réactif aux ions multivalents tel qu'un alginate d'alcalin, une géllane ou une pectine.

6. Procédé selon la revendication 5, **caractérisé en ce que** le polyélectrolyte réactif aux ions est un alginate d'alcalin ayant avantageusement une teneur en bloc α-L-guluronate supérieure à 50%, notamment supérieure à 55%.

7. Procédé selon l'une des revendications 5 ou 6, **caractérisé en ce que** la teneur massique en polyélectrolyte dans la deuxième solution (40) est inférieure à 5% en masse et est avantageusement comprise entre 0,5 et 3% en masse.

8. Procédé selon l'une quelconque des revendications précédentes, **caractérisé en ce que** la première solution (36) comprend l'un au moins d'un produit biologiquement actif, d'un produit cosmétique ou d'un produit comestible propre à être consommé.

9. Série de capsules (10), chaque capsule (10) comprenant un coeur liquide (12) contenant au moins un premier produit, et une enveloppe gélifiée (14) encapsulant totalement le coeur liquide (12) à sa périphérie, l'enveloppe gélifiée (14) étant propre à retenir le coeur liquide (12) lorsque la capsule (10) est plongée dans un gaz, l'enveloppe gélifiée (14) comprenant au moins un polyélectrolyte gélifié ;
**caractérisée en ce que** l'enveloppe gélifiée (14) comprend en outre au moins un agent tensioactif, la teneur massique en agent tensioactif dans l'enveloppe gélifiée (14) étant supérieure à 0,001%, et **en ce que** chaque capsule (10) présente une enveloppe gélifiée (14) ayant une épaisseur comprise entre 0,1% et 10%, avantageusement entre 0,1% et 2% du diamètre de la capsule (10).

## Patentansprüche

1. Verfahren zur Herstellung einer Reihe von Kapseln (10), wobei jede Kapsel (10) einen flüssigen Körper (12), der mindestens ein erstes Produkt enthält, und eine Gelhülle (14), die das Herz (12) vollständig einkapselt, umfasst, wobei das Verfahren die folgenden Schritte umfasst:
- separates Befördern in einer doppelten Hülle (32) einer ersten flüssigen Lösung (36), die das erste Produkt enthält, und einer zweiten flüssigen Lösung (40), die einen flüssigen gelierfähigen Polyelektrolyten enthält,
- Bilden, am Ausgang (50) der doppelten Hülle (32), einer Reihe von Tropfen (78), wobei jeder Tropfen (78) einen zentralen Kern (80), der von erster Lösung (36) gebildet ist, und eine periphere Membran (82), die von zweiter Lösung gebildet ist und den zentralen Kern (80) vollständig umschließt, umfasst,
- Eintauchen jedes Tropfens (78) in eine Gelierlösung (70), die ein Reagenz enthält, das imstande ist, mit dem Polyelektrolyten der Membran (82) zu reagieren, damit dieser aus einem flüssigen Zustand in einen gelierten Zustand wechselt und die Gelhülle (14) bildet, wobei der zentrale Kern (80) das flüssige Herz (12) bildet,
- Rückgewinnen der gebildeten Kapseln (10), in welchen die durch Coextrusion in der doppelten Hülle (32) gebildeten Tropfen (78) aufgrund von Schwerkraft durch ein Luftvolumen in die Gelierlösung (70) fallen,
**dadurch gekennzeichnet, dass** die zweite Lösung (40) vor ihrem Kontakt mit der ersten Lösung (36) mindestens ein Tensid enthält, und dass das Verhältnis des Durchsatzes der ersten Lösung (36) zum Durchsatz der zweiten Lösung (40) am Ausgang der doppelten Hülle (32) zwischen 1 und 200, in vorteilhafter Weise zwischen 10 und 200, inklusive ist, wobei die Gelhülle (14) nach Rückgewinnung der gebildeten Kapseln eine Stärke zwischen 0,1 % und 10 %, in vorteilhafter Weise zwischen 0,1 % und 2 %, inklusive des Durchmessers der Kapsel (10) hat.

2. Verfahren nach Anspruch 1, **dadurch gekennzeichnet, dass** das oder jedes Tensid aus einem anionischen Tensid, einem kationischen Tensid, einem nichtionischen Tensid oder ihren Gemischen ausgewählt ist.

3. Verfahren nach Anspruch 2, **dadurch gekennzeichnet, dass** das Tensid aus einem Alkylsulfat, einem Alkylsulfonat, einem Alkylarylsulfonat, einem alkalischen Alkylphosphat, einem Dialkylsulfosuccinat, einem erdalkalischen Salz gesättigter oder nicht gesättigter Fettsäuren, einem Halogenidsalz von Alkylpyridium oder von Alkylammonium wie dem n-Ethyldodecylammoniumchlorid oder -bromid, dem Cetylamoniumchlorid oder -bromid, den Polyoxyethylen- und/oder Polyoxypropylenderivaten der Fettalkohole, der Fettsäuren oder der Alkylphenole oder aus den Arylphenolen, den Alkylglucosiden, den Polysorbaten, den Cocamiden oder ihren Gemischen ausgewählt ist.

4. Verfahren nach Anspruch 3, **dadurch gekennzeichnet, dass** der Gewichtsprozentsatz insgesamt an Tensid in der zweiten Lösung (40) über 0,01 % ist und in vorteilhafter Weise zwischen 0,01 und 0,5 Gew.-% inklusive beträgt.

5. Verfahren nach einem der vorangehenden Ansprüche, **dadurch gekennzeichnet, dass** der oder jeder Polyelektrolyt ein mit mehrwertigen Ionen reagierender Polyelektrolyt ist, insbesondere ein mit mehrwertigen Ionen reagierendes Polysaccharid wie ein Alkalialginat, ein Gellan oder ein Pektin.

6. Verfahren nach Anspruch 5, **dadurch gekennzeichnet, dass** der mit Ionen reagierende Polyelektrolyt ein Alkalialginat ist, das in vorteilhafter Weise einen Blockgehalt α-L-Guluronat von über 50 %, insbesondere von über 55 %, hat.

7. Verfahren nach einem der Ansprüche 5 oder 6, **dadurch gekennzeichnet, dass** der Gewichtsgehalt an Polyelektrolyt in der zweiten Lösung (40) unter 5 Gew.-% ist und in vorteilhafter Weise zwischen 0,5 und 3 Gew.-% ist.

8. Verfahren nach einem der vorangehenden Ansprüche, **dadurch gekennzeichnet, dass** die ersten Lösung (36) mindestens ein Produkt eines biologisch aktiven Produktes, eines kosmetischen Produkts oder eines essbaren Produkts, das für den Verzehr geeignet ist, umfasst.

9. Reihe von Kapseln (10), wobei jede Kapsel (10) ein flüssiges Herz (12), das mindestens ein erstes Produkt enthält, und eine Gelhülle (14), die das flüssige Herz (12) an seiner Peripherie vollständig einkapselt, umfasst, wobei die Gelhülle (14) imstande ist, das flüssige Herz (12) zurückzuhalten, wenn die Kapsel (10) in ein Gas getaucht wird, wobei die Gelhülle (14) mindestens einen Gel-Polyelektrolyten umfasst,
**dadurch gekennzeichnet, dass** die Gelhülle (14) ferner mindestens ein Tensid umfasst, wobei der Gewichtsgehalt an Tensid in der Gelhülle (14) über 0,001 % ist, und dass jede Kapsel (10) eine Gelhülle (14) aufweist, die eine Stärke zwischen 0,1 % und 10 %, in vorteilhafter Weise zwischen 0,1 und 2 %, inklusive des Durchmessers der Kapsel (10) hat.

## Claims

1. A method for manufacturing a capsule series (10), each capsule (10) comprising a liquid body (12) containing at least one first product and a gelled casing (14) completely encapsulating the core (12), the method comprising the following steps:
- separately conveying, in a double casing (32), a first liquid solution (36) containing the first product and a second liquid solution (40) containing a liquid polyelectrolyte able to gel ;
- forming, at the outlet (50) of the double casing (32), a series of drops (78), each drop (78) comprising a central core (80) formed from the first solution (36) and a peripheral film (82) formed from the second solution and completely covering the central core (80);
- immersing each drop (78) in a gelling solution (70) containing a reagent able to react with the polyelectrolyte of the film (82) to make it go from a liquid state to a gelled state and form the gelled casing (14), the central core (80) forming the liquid core (12);
- recovering the capsules (10) formed, wherein the drops (78) formed by co-extrusion in the double casing (32) fall by gravity through a volume of air into the gelling solution (70);
**characterized in that** the second solution (40) contains at least one surfactant before the former contacts the first solution (36) and **in that** the ratio of the flow rate of the first solution (36) to the flow rate of the second solution (40) at the outlet of the double casing (32) is between 1 and 200, advantageously between 10 and 200, the gelled casing (14) having a thickness between 0.1% and 10%, advantageously between 0.1% and 2% of the diameter of the capsule (10), after recovery of the capsules formed.

2. The method according to claim 1, **characterized in that** the or each surfactant is chosen among an anionic surfactant, a cationic surfactant, a non-ionic surfactant, or mixtures thereof.

3. The method according to claim 2, **characterized in that** the surfactant is chosen from among an alkyl sulfate, an alkyl sulfonate, an alkyl aryl sulfonate, an alkaline alkyl phosphate, a dialkyl sulfosuccinate, a salt of saturated or unsaturated alkaline earth fatty acids, a salt of alkylpyridinium or alkylammonium halide such as n-ethyl-dodecylammonium chloride or bromide, cetylammonium chloride or bromide, polyoxyethylenated and/or polyoxypropylenated derivatives of fatty alcohols, fatty acids, or alkylphenols, or among arylphenols, alkyl glucosides, polysorbates, cocamides, or mixtures thereof.

4. The method according to claim 3, **characterized in that** the total weight percentage of surfactant in the second solution (40) is greater than 0.01% and is advantageously between 0.01% and 0.5% by mass.

5. The method according to any one of the preceding claims, **characterized in that** the or each polyelectrolyte is a polyelectrolyte reactive to multivalent ions, in particular a polysaccharide reactive to multivalent ions such as an alginate alkaline, a gellan, or a pectin.

6. The method according to claim 5, **characterized in that** the ion-reactive polyelectrolyte is an alginate alkaline advantageously having an α-L-guluronate block content greater than 50%, in particular greater than 55%.

7. The method according to one of claims 5 or 6, **characterized in that** the weight percentage of polyelectrolyte in the second solution (40) is less than 5% by mass and is advantageously between 0.5 and 3% by mass.

8. The method according to any one of the preceding claims, **characterized in that** the first solution comprises (36) at least one of a biologically active product, a cosmetic product, or a comestible product suitable for consumption.

9. A capsule series (10), each capsule (10) comprising a liquid core (12) containing at least one first product, and a gelled casing (14) completely encapsulating the liquid core (12) at the periphery thereof, the gelled casing (14) being able to retain the liquid core (12) when the capsule (10) is submerged in a gas, the gelled casing (14) comprising at least one gelled polyelectrolyte;
**characterized in that** the gelled casing (14) also comprises at least one surfactant, the weight percentage of surfactant in the gelled casing (14) being higher than 0.001%, and **in that** each capsule (10) has a gelled casing (14) with a thickness between 0.1% and 10%, advantageously between 0.1% and 2% of the diameter of the capsule (10).
